# EUROPEAN PATENT SPECIFICATION

(11) **EP 2 691 769 B1**
(45) Date of publication and mention of the grant of the patent: **21.01.2015**
(21) Application number: 12713555.6
(22) Date of filing: 30.03.2012
(51) Int. Cl.: G01N 33/483, A23J 1/00, A23L 1/212

(54) **ARTIFICIAL FECES**
KÜNSTLICHE FÄKALIEN
MATIERES FECALES ARTIFICIELLES

(30) Priority: 31.03.2011 US 201161470219 P; 16.03.2012 US 201213421925
(43) Date of publication of application: 05.02.2014
(73) Proprietor: The Procter & Gamble Company, Cincinnati, OH 45202 (US)
(72) Inventor: QIN, Wendy, Mason, Ohio 45040 (US); MARSH, Randall, Glenn, Hamilton, Ohio 45011 (US)
(74) Representative: Mather, Peter Geoffrey
(86) International application number: PCT/US2012/031348
(87) International publication number: WO 2012/135567

(56) References cited:
- WO-A1-01/52663
- WO-A1-2004/110176
- US-A- 3 433 650
- US-B1- 6 309 681
- DATABASE GNPD [Online] Mintel; January 2010 (2010-01), Anonymous: "Giant Tomato & Mozzarella Ravioli", XP002679265, retrieved from www.gnpd.com Database accession no. 1240525
- DATABASE GNPD [Online] Mintel; February 2011 (2011-02), Anonymous: "Hot Chorizo", XP002679266, retrieved from www.gnpd.com Database accession no. 1486661
- DATABASE GNPD [Online] Mintel; September 2008 (2008-09), Anonymous: "Greek Sesame Pizza", XP002679267, retrieved from www.gnpd.com Database accession no. 971174
- DATABASE GNPD [Online] Mintel; September 2005 (2005-09), Anonymous: "Chicken, Spinach & Cream Cheese Agnolotti", XP002679268, retrieved from ww.gnpd.com Database accession no. 396199

## Description

### FIELD OF THE INVENTION

This disclosure relates generally to compositions suitable for use as artificial feces.

### BACKGROUND OF THE INVENTION

Sometimes it is desirable to understand the chemical and/or physical behavior of feces. For example, it may be desirable to investigate how to remove feces from clothing or carpeting, as when toilet training a child or house breaking a puppy. It may be desirable to experiment with different methods or products for removing feces from hard surfaces, such as toilets or floors. It may also be desirable to evaluate how feces is handled by an absorbent article, such as a diaper, or removed from the skin, as with a personal cleansing wipe or toilet paper.

Investigators engaged in such work would generally prefer not to use real feces in experiments. Real feces, whether human or animal, may be capable of transmitting disease. Further, the smell of some feces samples, cultural norms, and other factors may make handling feces repugnant even when it is handled in a manner which minimizes the risk of disease transmission.

Some artificial feces alternatives have been proposed, and even sold commercially. However, when these products are used experimentally, the results may be inconsistent with experiments run with real feces, qualitatively or quantitatively. The value of the work done with such formulas may be reduced, because it remains uncertain whether the results of the study using artificial feces will be applicable when using real feces. Further, as a biological product, real feces is not a consistent substance. However, most artificial feces formulas are targeted to a single "type" of feces, and still only mimic a handful of the aspects of that feces type.

There remains a need for artificial feces.

### SUMMARY OF THE INVENTION

In some aspects, the present disclosure relates to a composition useful as artificial feces. In some aspects, the present disclosure relates to the use of an artificial feces to evaluate cleaning products or regimens. Additional aspects and various non-limiting embodiments are described in the Detailed Description of the Invention, below.

### BRIEF DESCRIPTION OF THE DRAWINGS

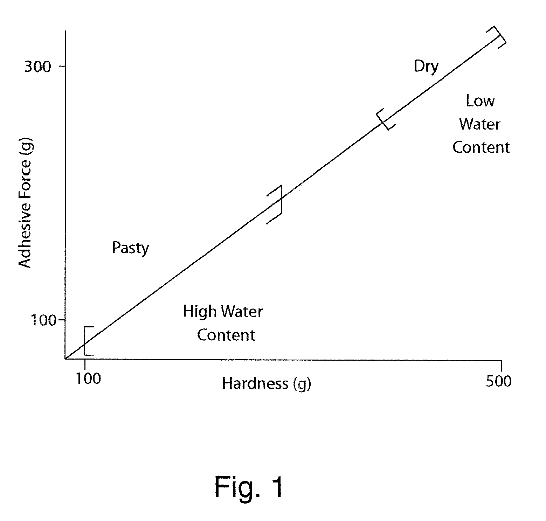

Fig. 1 is a plot of adhesive force (in grams) versus hardness (in grams) for exemplary artificial feces formulas of varying water content.

### DETAILED DESCRIPTION OF THE INVENTION

In some aspects, this disclosure relates to an artificial feces, which may also be referred to as synthetic feces or fecal simulant. In some aspects, this disclosure relates to a simulated soil with a quantifiable marker that can be non-invasively measured on a surface. The embodiments described below are referred to as artificial feces, however, it should be understood that the formulas may be adapted to simulate other soils, such as food wastes, vomitus, humus, and the like. The artificial feces may have solid components, which may include dried vegetable(s), fiber, yeast, yeast derivatives, and/or fatty acids. The artificial feces may have liquid components, which may include water, a preservative, and/or a mucus material. The artificial feces may be useful, for example, in studying waste handling or removal, including cleaning and stain removal, without the malodor or risk of disease transmission associated with handling real feces.

In some embodiments, an artificial feces comprises dried vegetables. Suitable vegetables include, but are not limited to, tomato, spinach, cabbage, okra, white (button) mushroom, carrot, pumpkin, and combinations thereof. The vegetable, or the predominant vegetable, if multiple vegetables are used, may be selected based on the intended use of the artificial feces. For example, the vegetables may fluoresce at different wavelengths of visible light with different intensities. For example, white mushroom, carrot, tomato, pumpkin, spinach, and cabbage powders are all visible as red fluorescence under green light. However, white mushroom, cabbage, carrot, and pumpkin may have more intense (and therefore more easily visible) fluoresce than tomato and spinach. In contrast, tomato, cabbage, and spinach provide bright green fluorescence under red light, and carrot provides a bright blue fluorescence under blue light. Under black light, white mushroom, tomato, carrot, cabbage, and pumpkin powders are easily visible, but spinach is harder to see with the unaided eye. Thus, the vegetable or the proportions of various vegetables in the artificial feces may be selected such that the artificial feces can be viewed under a specific lighting condition. The total vegetable content of the artificial feces may be from about 15% to about 35% of the solid content of the artificial feces.

The vegetable or predominant vegetable may also be selected for relative content of Adenosine TriPhosphate (ATP). ATP is a compound ubiquitous in living things, however, not all living things produce like quantities of ATP. The following processed food products are listed in decreasing order of typical ATP content: Tomato, peaches, sweet peas, beef, spinach powder, ham, cabbage, pumpkin, and carrot. If the artificial feces comprises ATP, it may be possible to track residues on a test surface using ATP quantification. For example, the artificial feces may be tested in advance for ATP content, in a known quantity of artificial feces applied to a test surface. The surface may then be cleaned or rinsed, and the surface tested for ATP. The quantity of ATP on the surface may be directly attributed to the artificial feces, for example, if the test surface is an inanimate object. Relative amounts of ATP may be used to infer the amount of artificial feces on animate surfaces, such as living skin.

In some embodiments, the artificial feces comprises a source of fiber. Exemplary sources of fiber include, but are not limited to, psyllium husks, processed foods, α-cellulose, cellulose derivatives including methyl-cellulose, corn starch, and combinations thereof. If processed foods are used, the food product may be selected for high fiber content. The food product may be, for example, ground, boiled, diced, shredded, lyophilized, sieved, suspended (as in a liquid) or combinations thereof. If a processed food is used, alone or in combination with other materials, as the fiber source, the food may be selected for high fiber content, such as greater than 0.5 grams of fiber per tablespoon, or greater than 1.0 grams of fiber per ounce. Exemplary foods which may have a relatively high fiber content include raspberries, pears (including the skin), apples (including the skin), strawberries, bananas, oranges, dried figs, raisins, whole wheat pasta, pearled barley, bran flakes, oat bran, oatmeal, popcorn (popped), brown rice, rye bread, whole wheat bread, multigrain bread, sweet peas, lentils, black beans, lima beans, sunflower seed kernels (i.e., shelled sunflower seeds), almonds, pistachio nuts (i.e., shelled pistachios), pecans (i.e, shelled pecans), artichokes, broccoli, turnip greens, sweet corn, Brussels sprouts, potatoes (including the skin), tomato paste, raw carrots, or combinations thereof. The fiber may provide bulk and water retention to the artificial feces. Less fiber may give an artificial feces with less cohesion than an artificial feces with relatively more fiber. To simulate soft, somewhat runny infant feces, the fiber may make up about 0.25% to about 1% of the solid content of the artificial feces. For other feces, the fiber may comprise up to 30% of the solid content of the artificial feces.

In some embodiments, the artificial feces may comprise yeast and/or yeast derivatives, such as hydrolyzed yeast autolysates. The yeast, if present, may be an additional source of ATP and contribute bulk to the artificial feces. Yeast products (whole cells and cell walls) may also provide proteins, polysaccharides, and other compounds which represent components of microbial origin in feces. Without wishing to be bound by theory, these elements may influence the surface chemistry, surface energy, and/or rheology of the artificial feces, and may, therefore, contribute to realistic "behavior" or characteristics. The yeast, if present, may be about 30% to about 50% of the solid content of the artificial feces. The yeast derivative, if present, may be about 10% to about 20% of the solid content of the artificial feces. Food grade yeast products may be used to represent proteins and microbial particles common in human feces. Live or inactivated yeast may be used, however, inactive yeast may produce a more stable intermediate product (i.e., during preparation), since the inactive yeast will not have on-going metabolic processes that might change ATP or protein content during processing.

In some embodiments, the artificial feces comprises a fatty acid and/or fatty acid soaps. If the artificial feces is used to simulate real feces, fatty acids typically found in the feces of the animal being studied may be used. The fatty acid may comprise palmitic acid, calcium-stearate, lauric acid, myristic acid, stearic acid, arachidic acid, behenic acid, lignoceric acid, cerotic acid, and combinations thereof. Sources of fatty acids may be used, including, but not limited to, cooking (vegetable) oil, Coffee Mate™ coffee creamer products, cream cheese, butter, peanut butter, ground sesame seed powder, and combinations thereof. If present, the fatty acid and/or fatty acid soap may be about 2% to about 50%, or about 15% to about 25% of the solid content of the artificial feces. The inclusion of one or more short chain fatty acids, having an aliphatic tail of less than six carbon atoms, may be used to enhance the realism of the artificial feces, as short chain fatty acids are a contributor to the odors associated with real feces. Realistic odor may be helpful in simulating human interactions with real feces. For example, an aversion to contacting real feces may change the way a person engages in a cleaning task. A real smell and/or appearance may help minimize study artifacts arising from researchers' and/or participants' awareness that the artificial feces is not real. Of course, other odorous compounds, or mixtures of fatty acids and/or other odorous compounds, can also be used, including, but not limited to, sulfur-containing compounds (such as hydrogen sulfide), nitrogen-containing compounds (such as amines), and certain aldehydes, ketones, alcohols, or any other compound having an unpleasant odor.

On the other hand, the artificial feces may seem quite realistic, and researchers or participants in a study may show some reluctance to handling the artificial feces. Social norms regarding the handling of feces are very strong, and even when the formula is intellectually recognized as artificial feces, it may still be unpleasant or "gross" to handle the artificial feces. In some embodiments, pigments may be added to make the artificial feces look like real feces. It is to be appreciated that pigments that are responsible for the color of real feces may be added to the artificial feces. For example, stercobilin, a pigment found in real feces, may be used. In some embodiments, dyes may be added to make the artificial feces look less like real feces. For example, non-limiting dyes which might be used in an artificial feces composition include food grade dyes, fluorescent dyes, water tracers (EPA-approved fluorescent dyes), colored or fluorescent beads (such as those commercially available as Glo-germ™ from DMA International, or Molecular Probes™ from Invitrogen), fluorescent tagged proteins, fluorescent tagged microbial particles, colored fruits and vegetables (such as beets, blueberries, or ground parsley flakes), similar colored or fluorescent materials, and combinations thereof. Instead of, or in addition to, dyes, fragrances might be used in an artificial feces composition. Like dyes, fragrances may signal that the composition is not real feces. Non-limiting fragrances which might be used in an artificial feces composition include essential oils (botanically-derived or synthetic), industrial perfumes or odor products, vegetable smells (synthetic or natural, including asparagus, bacon, peanuts, cheese, or combinations of these). In addition, the vegetable matter may be modified to include pleasant-smelling components, such as dried rosemary, dried sage, other dried herbs, dried flowers, or combinations thereof. Some people, however, may find pleasantly-scented artificial feces disconcerting, particularly if the artificial feces composition still looks very similar to real feces.

The solids may be ground into small particles. For example, the vegetable matter, fiber, and/or the yeast may be ground and sieved such that the nominal particle size is between about 100 and 500 microns, or between about 200 and 400 microns, or between about 250-300 microns. The vegetable, fiber, and/or yeast components may be sieved such that only particles which pass through a 300 micron sieve are used. If the solids are purchased as powders, it may be unnecessary to grind them, but may still be desirable to sieve them. Sieving the powders may contribute to more consistency across different batches of artificial feces. Smaller particles may make for a smoother composition, which may be suitable for mimicking, for example, the feces of a human infant. Larger particles or even small dices of dried vegetables may be used to simulate the feces of older animals, such as children or adults. Of course, a mix of particle sizes and morphologies, up to and including small cubes or chunks, could be used. If powdered or pre-ground products are purchased, attention should be paid to any additives. Anticaking agents, such as SiO2, may vary between products or between lots or batches of the same product, and can cause variation in the artificial feces. The content and/or consistency of the vegetable additives may be easier to control by purchasing dried vegetables whole, or in pieces or large dices, and grinding the vegetables just prior to use.

The artificial feces may comprise a liquid component. The liquid component may be predominantly water. If surface interactions and/or surface energy are of interest, deionized water may be used. Surfactants or other additives may also be used to vary the surface tension and/or surface energy of the artificial feces. The liquid component may comprise one or more preservatives, to reduce microbial growth in the artificial feces and enable a longer usage-life. Citric acid, which naturally arises in most animal feces both from food sources and as a by-product of the Krebs cycle, may be added to the water. Other acids, such as, phosphoric acid, acetic acid (vinegar), ascorbic acid, malic acid, oxalic acid, salicylic acid, organic acids, and combinations thereof may be used instead of or in addition to citric acid. It may be desirable to use one or more acids naturally present in foods, including fruits, vegetables, and drinks, such as soda and sports drinks. Fruit juices, soda, and vegetable drinks may also be used as acid sources. It may be desirable to adjust the pH until the artificial feces is inhospitable to many micro-organisms; a pH of less than 5, or approximately 4.6, has been found useful for this purpose. An acid, if used, may be present at a Molarity (moles/Liter) between 5x10⁻³ and 9x10⁻³. Of course, depending upon the formula, including any basic (alkaline) ingredients, the pKa of the acid being used, and the desired pH of the artificial feces, the desired Molarity of the acid or acids may vary within or outside this exemplary range. The liquid component may comprise benzyl alcohol. Benzyl alcohol is a naturally occurring substance in many plants, and may help inhibit microbial growth in the artificial feces between the time it is prepared and the time it is used. The artificial feces may comprise sodium benzoate. Sodium benzoate also occurs at low levels in dietary plants, and may help inhibit microbial growth in the artificial feces. In addition to, or instead of, benzyl alcohol and/or sodium benzoate, potassium sorbate, sorbic acid, or combinations thereof may be used. The primary purpose of the citric acid, benzyl alcohol, and/or sodium benzoate is to reduce microbial growth in the artificial feces; other preservatives or anti-microbials could be substituted for these compounds. Citric acid, benzyl alcohol, and/or sodium benzoate provide a preservative mixture which may be useful, for example, when the artificial feces may come in contact with human skin, because the compounds are naturally occurring, non-toxic, and at suitable concentrations, generally non-irritating. Substitute preservatives may be selected for similar properties, if desired. One or more acids may be desirable as a preservative or as a component of a preservative system because acids may lower the pH of the artificial feces, which both inhibits some microbial growth and helps preserve ATP in the triphosphate structure (i.e., maintain ATP counts over time).

The liquid component may comprise a mucus material, such as glycerol, egg white (fresh or dried), glycoproteins, polyacrylamides, polyvinylpyrrolidones, cellulose derivatives including methyl-cellulose and ethyl-cellulose, natural gums including gellan gum, polysaccharides, plants or plant exacts including okra, animal mucin including pig stomach mucin, combinations thereof, or other materials with similar adhesive and rheological properties. The mucus material, if present, may comprise between about 1% and about 20% of the liquid component of the artificial feces, by weight.

Although the artificial feces has been described as having solid and liquid components, it should be appreciated that these are not critical distinctions. As described in the examples herein, it may be convenient to relate proportions of components of the artificial feces to other elements in a like form, comparing, for example, grams of solids to grams of solids. However, many of the constituents described herein are available in multiple forms, including, for some compounds, solids, gels, liquids, suspensions, and the like, and the description should not be understood to limit the use of a particular compound to a particular form. It should be appreciated that proportions of compounds may be adjusted if the form used is changed (i.e., percent of solids vs. molarity of a liquid component, or to take into account the added liquid if using a suspension or solution rather than a powder). Accordingly, the descriptions of solid components and liquid components is convenient for describing certain examples, and is not meant to limit the form of the elements or the process for making the artificial feces.

One problem with some commercially available fecal simulants is that they do not retain water in the same way as real feces. Many fecal simulants dry much faster than real feces. This can be challenging in an experimental setting, because even freshly prepared artificial feces may dry out if there is a delay, for example, between applying an artificial soil and performing a cleaning step, or if the artificial soil is not quite tightly capped for even short periods of storage. This can also be a challenge if gravimetric measurements are taken, as time-to-testing can become a predictive variable for some previously known artificial feces formulas. Various humectants may be used to adjust the drying rate of artificial feces to make it easier to handle and/or to better simulate real feces. For example, glycerin, salts (such as sodium chloride), or sugars (such as sucrose), or combinations of these, may be used to retain water in the artificial feces to simulate water holding properties and the rate of evaporative drying in room temperature and humidity air (i.e., approximately 72-75°F and 40-60% relative humidity, or the typical temperature and humidity in the laboratory or room where the artificial feces will be used).

The artificial feces properties may be adapted by modifying the formula. ABM texture (hardness, adhesive force to standard control surface), can be modified by varying the water content of the artificial feces. In general, adding less water will make a harder artificial feces, and adding more water will make a runnier (lower hardness) artificial feces. For example, using the solid premix described below in Example 8, artificial feces described as dry can be prepared using a water content, for example, of 55 to 56% water, giving a composition with a hardness above about 325g. As another example, artificial feces described as pasty can be prepared using the solid premix described below in Example 8 and a water content, for example, of 63-64% water, giving a composition with a hardness between about 100 and about 250g. Artificial feces described as watery can be prepared using the solid premix described below in Example 8 and a higher water content, with a hardness below 50g and down to nearly 0g.

The distinction between "dry," "pasty," and "watery" artificial feces is based on groupings that may be helpful in simulating common human feces types; however, it should be appreciated that the texture can be varied predictably based on water content. For a given solid premix, such as the premix described in Example 1, if artificial feces of varying water content is graphed by adhesive force vs. hardness, there is a linear relationship, with lower water content correlated to higher adhesive forces and hardness relative to higher water content, as shown in Figure 1. Thus, it is possible to make not only dry, pasty, and watery artificial feces as described herein, but to modify the artificial feces to achieve various textures in between and beyond those groupings. For example, artificial feces harder than "dry" feces may correspond to some kinds of non-human animal feces or other soils, and may be prepared by reducing the water content of the artificial feces. Further, it should be understood that the water content guidelines provided are exemplary, based upon the solid premix of Example 1. Different solid premix formulas may have different water contents associated with different artificial feces textures.

One possible advantage of an embodiment comprising primarily (that is, 50% or more by weight, or 75% or more by weight) water and food-grade ingredients (such as dried vegetables and yeast) is that the artificial feces may be safe for use on human skin, including potentially sensitive human skin, such as infant skin, or facial or neck skin, or skin in the perineum or intertriginal regions of the thighs. The artificial feces may be applied to a surface, such as glass, or simulated skin (also referred to a skin mimic), or live skin *in vivo,* and then cleaned with a solvent, cleanser, and/or implement, and the residues remaining after cleaning can be viewed under non-standard lighting conditions that highlight the fluorescent residues. As a more specific example, artificial feces may be applied to human skin, such as a forearm, hand, leg, or even a baby's perineum, and the skin cleaned, as with a cleansing wipe, and it is possible to show any residues left behind by the cleansing wipe (or other product). Thus, cleaning regimens and/or products could be evaluated using this artificial feces in a qualitative way, looking for patterns of residual or observations of the relative amounts of residuals. As described above, some artificial feces may fluoresce under colored light, and thus it may be possible to visibly demonstrate soils left on a test surface after a cleaning procedure. The fluorescence may highlight soils not easily visible under standard lighting conditions, and allow for the observation of small differences in the effectiveness of different cleaning procedures.

Test surfaces may include, for example, human skin, animal skin, skin mimic material, glass, metal, tile, porcelain, other ceramic materials, stone, formica, terrazo, concrete, linoleum, carpet, wood, and combinations thereof. Exemplary situational test surfaces may include tiles, floors, carpets, toilet bowls, toilet seats, door knobs, counter tops, bathroom surfaces, furniture surfaces, body parts, and the like. Human or animal skin may be used as a test surface *in vivo,* or excised skin samples (live or from cadavers) may be used. Skin mimic materials may include polymers selected for some similarity to the skin of interest (whether human or animal, hairy or smooth, populated with sebaceous glands or not, etc.). For example, a polymer-based skin mimic may be selected for its surface energy; or textured to simulate the outermost layer of a skin surface; or shaped to simulate the topography of a skin surface *in situ*; or coated to simulate the surface energy, coefficient of friction, polarity, or other properties of the skin of interest. Some exemplary coatings are described, for example, in WO 2007/021844 to Belcher, et al.

Thus, artificial feces as described herein may be useful in a method for developing a cleaning product or procedure. The cleaning product may be a cleansing composition (including personal cleansing compositions, surface cleansing compositions, soaps, and detergent compositions), a wipe, a duster, a brush, a cloth, a towel, a sponge (including natural, synthetic, and gauze sponges), a paper towel, a napkin, toilet paper, or the like. Although water is often used with a cleansing product, water itself is not considered a "cleaning product" for the purpose of the present disclosure. A cleaning procedure may involve steps such as rinsing, wiping, and/or scrubbing a surface and/or contacting the surface with a cleaning product. In some aspects, the method may comprise identifying a test site on a surface. The test site may be evaluated for cleanliness. For example, the test site may be visualized using colored light imaging, or black light, and/or may be tested qualitatively or quantitatively for ATP. A fixed quantity of artificial feces may be applied to the test site. The artificial feces may comprise a known ATP count or quantity. The test site may be subjected to a cleaning procedure. The test site may be evaluated for cleanliness before and/or after being subjected to a cleaning procedure. More than one test site may be used on the same or similar surfaces. For example, the skin on the left anterior forearm of a single human test subject is considered the same surface, and the skin on the left anterior forearm and right anterior forearm of a single human test subject are considered similar surfaces, assuming the skin surfaces are normal, with no scars, injuries, or irritations prior to testing. Two or more test sites are on the "same surface" if they are on different portions of a single, reasonably homogenous, continuous surface, and two or more test sites are on "similar surfaces" if they are on different surfaces of the same kind, such as two glass plates of like composition, or two arms of the same human subject, etc. By reasonably homogenous, it is meant that the surface should not have avoidable variations in surface properties that may alter test results, it being recognized that a higher degree of variation may be unavoidably present in biological systems, such as skin or wood, than in inanimate surfaces, such as glass or metal test plates. If multiple test sites are used, the test sites may be subjected to different treatments. For example, one or more test sites may be untreated, to serve as a control. A control test site may be helpful particularly, but not exclusively, on live and/or biological test surfaces, such as skin or hair. One or more test sites may be washed with soap and water, which is considered a "gold standard" for cleanliness, and also serves as a control. If the soap and water wash does not maintain or improve cleanliness of the surface, there may be a problem with the experimental design, experimental execution, and/or data analysis. Other test sites may be treated with different cleaning products or procedures, or with different combinations of cleaning products and/or cleaning procedures. The cleanliness of differently treated test sites may be compared to evaluate possible changes to cleaning products and/or procedures.

ATP quantification may be performed using, for example, the AccuPoint sampling and reading system available from Neogen Corp, which allows direct surface sampling and uses a luciferase-luciferin enzyme system to convert ATP to radiant energy (i.e., light), which can be measured and quantified as Relative Light Units (RLUs). Any other analytical method suitable for quantifying ATP may be used, and different methods may be more or less desirable for different experimental designs. As further, non-limiting examples, ATP can be quantified by spectroscopy, mass spectrometry, HPLC with UV detection, luciferase-luciferin systems or other enzymatic tests such as those based on creatine kinase or ATP synthase, and ion exchange chromatography with UV detection.

In some embodiments, gravimetric methods may be used to evaluate soil transfer between a surface and a cleaning implement. For example, a controlled insult of known mass may be applied to a test surface. A cleaning product may be applied to the area of the controlled insult, as by placing the cleaning product (such as a wipe, paper towel, toilet paper, or the like) over the insult, or wiping the area of the controlled insult with the cleaning product, or otherwise contacting the area of controlled insult with the cleaning product. The cleaning product may be weighed before and after contact with the area of controlled insult. The difference between the mass of the cleaning product after contact with the area of controlled insult and the mass of the cleaning product before contact with the area of controlled insult can be attributed to the soil or simulated soil (from the controlled insult) which was removed by the cleaning product. An exemplary procedure for gravimetric analysis of the removal of soils from skin is described, for example, in U.S. Patent No. 7,744,531 to Marsh, et al.

It should be appreciated that depending upon the specific formula used, the artificial feces described herein may be detected using any number of other assays, including tests for specific proteins, DNA (as from vegetables and/or yeast), or compounds like lycopene, which may not be inherently present on the surface to be tested, yet appear in the artificial feces.

Similar steps may be useful in a method for demonstrating the efficacy of a cleaning product and/or procedure. For example, test sites subjected to different treatments may be compared under black light, or by colored light imaging, to visually demonstrate the residual artificial feces on the test site after treatment. As described above, the residual artificial feces may be difficult or impossible to see under typical lighting conditions (e.g., standard fluorescent lighting, standard incandescent lighting, sunlight). Thus, artificial feces which fluoresces under a given lighting condition, such as red light, or blue light, or green light, or black light, may be used to visually demonstrate the efficacy of a cleaning product and/or procedure, and/or to compare the efficacy of different cleaning products and/or procedures, even when the differences would not normally be visually observable under typical lighting conditions with the unaided human eye. Of course, analytical results, such as the presence and/or quantity of ATP remaining on a test site after a treatment could also be used to demonstrate efficacy. Depending, for example, upon the audience, a visual demonstration may be preferred to a data-based demonstration, or vice-versa.

The methods described above may also be useful vis-à-vis surfaces other than the surface being cleaned. For example, a participant in a study may be asked to clean a surface, and the hand (or other appendage) used to perform the cleaning may be tested. Some cleaning products and/or procedures may suitably clean a surface, but allow soils from the surface to transfer to the hand of the cleaner. As one more specific example, some wipe products may, under some conditions, transmit soils through the thickness of the wipe. Thus, testing the hand of the cleaner could show differences between cleaning products and/or procedures with regard to the cleaner's contact with soils while cleaning. In some embodiments, such cleaning testing may be automated (e.g., use a robotic "hand" or functionally equivalent apparatus rather than a human test participant). In such embodiments, it may be desirable to test the surface of the test apparatus equivalent to a cleaner's hand for contamination.

Because some of the formulas described may mimic with new fidelity the adhesion of feces to human skin, they may be useful in understanding the role of fecal residues in skin health and/or skin condition. For example, it was once believed that contacting the skin with urine produced diaper rash, however, it is now understood that the irritation of tissue which manifests itself in "diaper rash" is primarily caused by endogenic proteolytic and/or lipolytic enzymes, inter alia, trypsin, chymotrypsin, elastase, pancreatic lipase, which frequently occur in human feces.

Enzymes present in feces include proteolytic enzymes, lipases and other esterases and diesterases, ureases and other enzymes including amylases, elastases, nucleases, and the like. Although the relative contribution of the different types of enzymes to skin irritation is unknown, there is evidence that at least fecal proteolytic and lipolytic enzymes, of intestinal and/or pancreatic origin, play a direct role in causing the skin inflammation of diaper rash.

Studies with inhibitors designed to inhibit the enzymatic activity of various classes of proteases have shown that serine proteases, cysteine proteases and metalloproteases were the most likely to be responsible for the overall proteolytic activity of feces. It is known that the serine proteases trypsin and chymotrypsin, in particular, are nearly always present in grossly measurable quantities in the stools of normal young children, and smaller but detectable quantities are present in normal adult stools. Lipases, including esterases that hydrolyze dietary triglycerides, are also found in normal stools and are capable of hydrolyzing triglycerides and other glycerides found in human skin to form irritating fatty acid and glycerol by-products. Thus, when skin is exposed to enzymes such as lipases and proteases present in body exudates, lipid-containing components and protein-containing components of the skin, especially of the barrier layer (stratum corneum), can be broken down resulting in the irritation and inflammation of diaper rash. Moreover, perturbation of the skin barrier allows other components of urine and feces, ammonia, bacteria and the like which may not otherwise be irritating by themselves, to migrate through the compromised skin barrier to produce additional irritation and possible infection.

It is known that bile salts are also present in body exudates. These bile salts are known normally to emulsify lipids in the body to ensure that the lipase enzymes are capable of performing at the lipid/water interface. Bile salts are also active when excreted in feces and other exudates and are available to act as coenzymes and enhance the activity of lipases that attack lipids in the stratum corneum of the skin that is exposed to body exudates.

In some embodiments, an artificial feces composition as described herein may comprise fecal-relevant enzymes, as discussed above, or bile salts, or both. Such embodiments may be useful, for example, for studying the effect of various types and/or levels of fecal residues on the skin over various time periods, or for studying the effectiveness of compositions intended to counteract the effect of such fecal residues on the skin. Additional information about the relationship between fecal residues and skin health, and exemplary compositions comprising enzyme inhibitors, are described in U.S. Patent No. 6,703,536 to Roe, et al.

### Example 1-Preparation of dry powder mix ("Solid premix")

A solid premix was made according to the formula in Table 1, below. An IKA A11 basic grinder was used to grind the vegetables: dehydrated tomato dices (Harmony House or NorthBay); dehydrated spinach flakes (,Harmony House or NorthBay); dehydrated cabbage (Harmony House or NorthBay); whole psyllium husk (available from Now Healthy Foods, sieved with 600 µm cutoff to collect greater than 600 µm particles and then ground to collect 250-300 µm particles) (alternatively available from Barry Farm as a powder that has to be sieved to collect 250-300 µm particles); palmitic acid (95% Alfa Aeser B20322); and calcium stearate (Alfa Aeser 39423). The vegetable flakes were added to the grinding bowl, filled to the mark (within the metal cup, not over-filled). The grinder was powered on for 5 seconds, stopped, and the powder was tapped 5 times. The grinder was again powered on and tapped 4 times (i.e., a total of 5 cycles of powering on and tapping). The ground powder was sieved by stacking a 600 µm opening sieve on top of a 300 µm opening sieve such that powders of 300 µm or less were collected. Any remaining powders that are larger than 300 µm are re-ground one time. Powders of 300 µm or less were collected. Next add food grade yeast powders commercially available as Provesta^{®} 000 and Ohly^{®} Auxoferm HCT (both commercially available from Ohly Americas, Hutchinson, MN) were added. The palmitic acid/calcium steartate blend was prepared by grinding together and collecting powders of 300 µm or less from a blend of 20.0005 g palmitic acid and 10.006 g calcium stearate and added to the solid premix.

**Table 1**

| Soil Powder Premix | Grams | % |
|---|---|---|
| Tomato Powder | 20.059 | 18.353 |
| Psyllium Husk | 0.599 | 0.548 |
| Cabbage | 2.145 | 1.963 |
| Spinach Powder | 8.129 | 7.438 |
| Provesta 000 | 40.906 | 37.428 |
| Ohly HCT | 16.628 | 15.214 |
| Palmitic acid / Calcium Stearate (2:1) | 20.827 | 19.056 |

### Example 2-Preparation of Liquid Premix

A water premix was made by adding 0.7 mL 1M citric acid, 0.3 mL benzyl alcohol, and 0.125 g sodium benzoate to 70mL of distilled water. Glycerin (10g) was weighed in a separate container and added the water premix to bring total weight of water, preservatives and glycerin (i.e., the Liquid premix) to 10 times the weight of the glycerin alone (i.e., 100g).

### Example 3-Preparation of Watery Artificial Feces

To prepare the artificial feces, water premix, described above, was added to solid premix, described above in Table 1, in a suitable container, to achieve a water content between about 70% to about 80%. A tongue depressor was used to stir the composition until the composition, which may be a paste, is homogeneous. The container is capped loosely and covered with a piece of aluminum foil. The container was placed in a fully boiling steamer for 40 minutes. The container was removed from the steamer after the 40 minutes and allowed to cool down to 23° ± 2.2° C. The test composition was ready for use. If desired, transfer the test composition to a syringe using a sterile tongue depressor for ease of handling.

### Example 4-Preparation of Dry Artificial Feces

To prepare the artificial feces, water premix, described above, is added to solid premix, described above in Table 1, in a suitable container, to achieve a water content between about 50% to about 56%. A tongue depressor is used to stir the composition until the composition, which may be a paste, is homogeneous. Cap the container loosely and cover it with a piece of aluminum foil. Place the container in a fully boiling steamer for 40 minutes. Remove the container after the 40 minutes and let cool down to 23° ± 2.2° C. The test composition is ready for use. If desired, transfer the test composition to a syringe using a sterile tongue depressor for ease of handling. Covered, the Dry Artificial Feces is stable at room temperature for at least 5 days.

### Example 5-Preparation of Pasty Artificial Feces

To prepare the artificial feces, water premix, described above, is added to solid premix, described above in Table 1, in a suitable container, to achieve a water content between about 61% to about 65%. A tongue depressor is used to stir the composition until the composition, which may be a paste, is homogeneous. Cap the container loosely and cover it with a piece of aluminum foil. Place the container in a fully boiling steamer for 40 minutes. Remove the container after the 40 minutes and let cool down to 23° ± 2.2° C. The test composition is ready for use. If desired, transfer the test composition to a syringe using a sterile tongue depressor for ease of handling. Covered tightly, the Watery Artificial Feces is stable at room temperature for at least 5 days. By stable, it is meant that no appreciable change in ATP counts, hardness, adhesive force, or cohesiveness is expected.

### Example 6-Qualification of Artificial Feces for a Particular Purpose

Because animal feces, including human feces, varies widely with diet, age, and physical condition, it may be desirable to further modify one of the formulas described in examples 3-5 to produce a formula specific to a particular kind of feces. For example, adhesion (to a surface or material) can be qualified by collecting real feces of the kind of interest (e.g., watery), and testing the adhesion of the real feces to a standard material. The standard material may be a cleaning implement or a component of a cleaning implement, such as a nonwoven material. A spunbond nonwoven of 100% viscose, may, for example, be used. A known quantity of the real feces is placed on a controlled surface, such as a piece of weigh paper of known mass. The feces is turned over onto a standard sample, in this example a nonwoven material, and a downward pressure (for example, 0.5psi or equivalent) is exerted (as, for example, by a 500gram weight placed on top of the standard sample, feces, and weigh paper) for a fixed time period (for example, 10 seconds). The weight is removed and the weigh paper is peeled away from the standard sample. The standard sample is weighed before and after testing, making it possible to calculate the amount of real feces which adhered to the standard sample. The test can then be repeated with the Artificial Feces until an acceptable approximation is achieved. It may be desirable to use a dry standard material for this qualification testing, to reduce the influence of evaporative drying on the gravimetric measurements.

### Example 7

Hardness is measured using a Stevens-Farnell QTS-25 Texture Analyzer, model 7113-5 kg, and associated software on an Intel-based machine having a 486 processor or higher, or equivalent. A 1/2 inch stainless steel spherical probe and a sample receptacle are provided. A suitable probe is the TA18 probe available from Leonard Farnell Co. of Hatfield, England. The sample receptacle can be made by cutting a 7 milliliter linear low density polyethylene scintillation vial (having an inside diameter of 0.55 inches .+-.0.005 inches) to a 15 millimeter length. Suitable vials are available from Kimble Glass Company of Vineland, N.J. as #58503-7 vials. The sample receptacle is filled to within 2 millimeters of the top edge with the sample to be tested. The vial is centered under the 1/2 inch spherical stainless steel probe. The probe is lowered to a distance of about 1 millimeter from the surface of the sample in the vial. The probe 162 is moved downward 7 millimeters at 100 millimeters per minute and then stopped. The Hardness is the maximum recorded resistive force encountered by the probe on its 7 millimeter downstroke. The temperature of the room and the analog should be between about 65 to 75 degrees Fahrenheit during the course of the measurement.

### Example 8

A solid pre-mix has the following components (in weight percent):

| | |
|---|---|
| Soil Powder Premix | % |
| Tomato Powder | 32.6 |
| Psyllium Husk | 2.7 |
| Spinach Powder | 10.8 |
| Provesta 000 | 32.5 |
| Ohly HCT | 6.4 |
| Palmitic acid / Calcium Stearate (2:1) | 15.0 |

Some embodiments described herein may present particular advantages. For example, a formula as described herein may have an appearance that is similar to real feces, such that the formula may be useful for testing the ability to remove stains (as with a cleaning product) or to hide or disguise feces (as in an absorbent article). A formula as described herein may have a surface energy similar to real feces, such that cleaning wipes or other implements are likely to adhere (or to not adhere) to the formula as they would to real feces. A formula as described herein may further have rheological properties similar to real feces, such that the formula is useful for testing the removal of soil from hard or soft surfaces, such as a toilet bowl or bathroom floor, or the skin of a baby or adult human or animal.

## Claims

1. A method for comparing the efficacy of a cleaning product or procedure, the method comprising:
identifying a test site;
applying a known quantity of artificial feces to the test site, the artificial feces comprising one or more dried vegetables, fiber, yeast or yeast derivatives, a fatty acid and/or fatty soap, and water;
cleaning the test site; and
evaluating the cleanliness of the test site using fluorescence visualization, Adenosine TriPhosphate (ATP) quantification, or gravimetric analysis.

2. The method according to Claim 1 wherein two or more cleaning products or procedures are tested using two or more test sites.

3. The method according to Claim 2, wherein a difference in efficacy between the two or more cleaning products or procedures is evaluated visually or by imaging.

4. The method according to Claim 1, 2, or 3 wherein the test site is disposed on a surface, and the surface is selected from the group consisting of human skin, animal skin, skin mimic material, glass, metal, tile, porcelain, other ceramic materials, stone, formica, terrazo, concrete, linoleum, carpet, wood, and combinations thereof.

## Patentansprüche

1. Verfahren zum Vergleichen der Wirksamkeit eines Reinigungsprodukts oder -Verfahrens, wobei das Verfahren Folgendes umfasst:
Bestimmen einer Teststelle,
Auftragen einer bekannten Menge von künstlichen Fäkalien auf die Teststelle, wobei die künstlichen Fäkalien ein oder mehrere Trockengemüse, Faser, Hefe oder Hefederivate, eine Fettsäure und/oder Fettseife und Wasser umfassen,
Reinigen der Teststelle und
Bewerten der Reinheit der Teststelle mittels Fluoreszenzdarstellung, quantitativer Bestimmung von Adenosintriphosphat (ATP) oder gravimetrischer Analyse.

2. Verfahren nach Anspruch 1, wobei zwei oder mehr Reinigimgsprodukte oder -verfahren unter Verwendung von zwei oder mehr Teststellen getestet werden.

3. Verfahren nach Anspruch 2, wobei ein Wirksamkeitsunterschied zwischen den zwei oder mehr Reinigungsprodukten oder -verfahren visuell oder durch Bildgebung bewertet wird.

4. Verfahren nach Anspruch 1, 2 oder 3, wobei die Teststelle auf einer Oberfläche angeordnet ist und die Oberfläche ausgewählt wird aus der Gruppe bestehend aus menschlicher Haut, Tierhaut, hautimitierendem Material, Glas, Metall, Fliese, Porzellan, anderen Keramikmaterialien, Stein, Resopal, Terrazo, Beton, Linoleum, Teppich, Holz und Kombinationen davon.

## Revendications

1. Procédé pour comparer l'efficacité d'un produit ou d'une procédure de nettoyage, le procédé comprenant :
identifier un emplacement d'essai ;
appliquer une quantité connue de selles artificielles sur l'emplacement d'essai, les selles artificielles comprenant un ou plusieurs légumes secs, des fibres, de la levure ou des dérivés de levure, un acide gras et/ou un savon gras, et de l'eau ;
nettoyer l'emplacement d'essai ; et
évaluer la propreté de l'emplacement d'essai en utilisant la visualisation par fluorescence, la quantification d'adénosine triphosphate (ATP), ou l'analyse gravimétrique.

2. Procédé selon la revendication 1, dans lequel deux produits ou procédures de nettoyage ou plus sont testés en utilisant deux emplacements d'essai ou plus.

3. Procédé selon la revendication 2, dans lequel une différence d'efficacité entre les deux produits ou procédures de nettoyage ou plus est évaluée visuellement ou par imagerie.

4. Procédé selon la revendication 1, 2 ou 3, dans lequel l'emplacement d'essai est disposé sur une surface, et la surface est choisie dans le groupe constitué par la peau humaine, la peau d'animal, un matériau imitant la peau, le verre, le métal, la céramique, la porcelaine, d'autres matériaux céramiques, la pierre, le formica, le terrazzo, le béton, le linoléum, les tapis, le bois, et leurs combinaisons.
